# EUROPEAN PATENT APPLICATION

(11) **EP 1 788 092 A1**
(43) Date of publication of application: **23.05.2007**
(21) Application number: 05734457.4
(22) Date of filing: 19.04.2005
(51) Int. Cl.: C12Q 1/02, A61K 31/185, A61K 31/198, A61K 31/245, A61K 31/26, A61K 38/43, A61K 45/00, A61P 9/00, A61P 9/04, A61P 9/10, A61P 25/16, A61P 25/28, A61P 43/00, C12Q 1/37, C12Q 1/68, G01N 33/15, G01N 33/50

(54) **METHOD OF INHIBITING POSTISCHEMIC REPERFUSION-INDUCED CELL DEATH AND CELL DEATH INHIBITOR**

(30) Priority: 30.07.2004 JP 2004224884
(71) Applicant: Japan Science and Technology Agency, Kawaguchi-shi, Saitama 332-0012 (JP); National Institutes of Natural Sciences, Tokyo (JP)
(72) Inventor: OKADA, Yasunobu, Okazaki-shi, Aichi 444-0877 (JP); TAKAHASHI, Nobuyuki, Okazaki-shi, Aichi 444-0831 (JP)
(74) Representative: Graf von Stosch, Andreas
(86) International application number: PCT/JP2005/007479
(87) International publication number: WO 2006/011273

(57) **Abstract**

It is intended to provide a method of preventing or relieving reperfusion-induced cell death (apoptotic cell death) of myocardial cells due to postischemic reperfusion; a reperfusion-induced cell death (apoptotic myocardial cell death) inhibitor; and therapeutic drug or a preventive drug for diseases caused by reperfusion-induced cell death (apoptotic cell death) which contains the reperfusion-induced cell death inhibitor. Namely, reperfusion-induced cell death (apoptotic cell death) can be significantly inhibited by administering a VSOR Cl channel blocker or an ROS scavenger to myocardial cells, which have been subjected to an ischemic treatment,. during reperfusion. As the VSOR Cl channel blocker, it is preferable to use, for example, DIDS or NPPB. As the ROS scavenger, it is preferable to use, for example, Tiron, catalase or NAC. Thus, various diseases caused by reperfusion-induced cell death can be effectively treated or prevented.

## Description

### TECHNICAL FIELD

The present invention relates to (i) a method for inhibiting a cell death, especially apoptotic cell death induced by postischemic reperfusion due to bloodstream injury in myocardial cells, to (ii) a cell death inhibitor, (iii) a therapeutic drug and a preventive drug for diseases caused by the cell death. Further, the present invention relates to a method for evaluating an inhibitory effect on the apoptotic cell death induced by postischemic reperfusion.

### BACKGROUND ART

It is well known that apoptosis in myocardial cells plays an important role in onset and exacerbation of myocardial and vascular diseases (see Non-Patent Citations 1 and 2, for example). Further, it was proved that apoptosis is the causes for most of cardiac cell deaths observed in parts where post-cardiac ischemia reperfusion injury or hypoxia/reoxygenation injury occurred (see Non-Patent Citations 3 to 5). Efforts to develop drugs for preventing or inhibiting the apoptotic cell death in myocardial cell which are reperfused after ischemia have been failed to provide satisfactory one.

It is known that cell shrinkage occurs in an initial stage of apoptosis. The inventors of the present invention proved that KCl flow-out due to acts of Cl- channel and K⁺ channel relates to a cause of apoptotic volume decrease (AVD) in apoptosis induced by staurosporin (STS), which is a mitochondria-mediating stimulus or by tumor necrosis factor-α (TNF α), which is a death receptor intervening stimulus (see Non-Patent Citation 6).

The inventors of the present invention developed a method for remedying apoptotic cell death by administering a drug for blocking the act of the Cl- channel (see Patent Citation 1).

Furthermore, the inventors of the present invention developed a method for screening for a therapeutic drug or a preventive drug for a cardiac disease by utilizing a system in which apoptosis is induced in cultured myocardial cells or vascular endothelial cells by STS and then remedied by administrating a Cl- channel blocker (inhibitor) (for example, see Patent Citation 8).

After that, it was reported that apoptotic cell death in myocardial cell in ectopic transfer of a rat heart can be inhibited by the Cl- channel blocker (administered before extirpation of a heart and after transplantation of the heart) (see Non-Patent Citation 7).

However, a method has not been developed, which prevents or remedies so-called postischemic reperfusion-induced apoptotic cell death (not at the time of ischemia) by this strategy, namely, glucose re-administration and reoxygenation to myocardial cells after oxygen glucose deprivation (OGD) of the myocardial cells.

It is known that reactive oxygen spices (ROS) relate to the occurrence of the postischemic reperfusion-induced apoptosis in myocardial cells (see Non-patent Citation 8).

Recently, the inventors of the present invention proved that volume-sensitive outwardly rectifying Cl- channel: VSOR Cl-channel) is activated at AVD in the initial stage of the apoptosis either in the case of mitochondria stimulus by STS or in the case of death receptor stimulus by using TNF (Tumor Necrosis Factor-α) or Fas ligand. Thereby, the inventors showed that the activation signal to the channel in the case of the mitochondria stimulus is ROS (see Non-Patent Citation 9).

Meanwhile, several methods have been developed, which inhibiting cardiac disease and blood vessel injury by preventing ROS production (for example, see Patent Citations 2 to 7). However, no method has been developed, which prevents or remedies the postischemic reperfusion-induced myocardial cell apoptosis cell death by eliminating ROS.

Moreover, Patent Citation 9 discloses (I) use of a compound (such as histamine etc.) that inhibits production of Reactive Oxygen Metabolite (hereinafter, referred to as "ROM") can inhibit and reduce ROM-induced diseases; (II) if necessary, an ROM scavenger (such as catalase etc.) that captures ROM is used in addition to the compound that inhibits the ROM production; (III) Some examples of the ROM-inducing diseases is ischemia/reperfusion injury, and one specific example thereof is stroke. However, the disclosures (I) to (III) are mere suggestions that predict the possibility of inhibiting "ROM-induced diseases" by "inhibiting the ROM production" or "capturing the produced ROM". As described later, the evaluation of the effect of inhibiting the reperfusion-induced cell death is not known as per filing of the present application. Thus, it is not possible for a person skilled in this art to arrive the present invention without a means for examining the suggestions presented by Patent Citation 8.

In addition, Patent Citation 10 describes that a novel salen-metal composite containing manganese; cobalt; copper; iron; etc. has an anti-oxidative effect and is effective in remedying myocardial infarction.
Non-Patent Citation 1
   W. R. MacLellan & M. D. Schneider "Death by design. Programmed cell death in cardiovascular biology and disease." Circ. Res. 81, 137-144, 1997
Non-Patent Citation 2
   A. Haunstetter & S. Izumo "Apoptosis: basic mechanisms and implications for cardiovascular disease." Circ. Res. 82, 1111-1129, 1998
Non-Patent Citation 3
   R. A. Gottlieb, K. O. Burleson, R. A. Kloner, B. M. Babior & R. L. Engler "Reperfusion injury induces apoptosis in rabbit cardiomyocytes." J. Clin. Invest. 94, 1621-1628, 1994
Non-Patent Citation 4
   H. Fliss & D. Gattinger "Apoptosis in ischemic and reperfused rat myocardium." Circ. Res. 79, 949-956, 1996
Non-Patent Citation 5
   A. Elsasser, K. Suzuki, S. Lorenz-Meyer, C. Bode & J. Schaper "The role of apoptosis in myocardial ischemia: a critical appraisal." Basic Res. Cardiol. 96, 219-226, 2001
Non-Patent Citation 6
   E. Maeno, Y. Ishizaki, T. Kanaseki, A. Hazama & Y. Okada "Normotonic cell shrinkage due to disordered volume regulation is an early prerequisite to apoptosis." Proc. Natl. Acad. Sci. USA 97, 9487-9492, 2000
Non-Patent Citation 7
   K. Mizoguchi, H. Haeta, A. Yamamoto, M. Oe & H. Kosaka "Amelioration of myocardial global ischemia/reperfusion injury with volume-regulatory chloride channel inhibitors in vivo." Transplantation 73, 1185-1193, 2002
Non-Patent Citation 8
   D. Kumar & B. I. Jugdutt "Apoptosis and oxidants in the heart." J. Lab. Clin. Med. 142, 288-297, 2003
Non-Patent Citation 9
   T. Shimizu, T. Numata & Y. Okada "A role of reactive oxygen species in apoptotic activation of volume-sensitive Cl-channel." Proc. Natl. Acad. Sci. USA 101, 6770-6773, 2004)
Patent Citation 1
   Japanese Patent Application Publication, Tokukai, No. 2002-3402 (published on January 9, 2002)
Patent Citation 2
   Japanese Translation Publication of International Application, Tokuhyo, No. 2003-505341 (published on February 12, 2003; and internationally published on October 12, 2000))
Patent Citation 3
   Japanese Translation Publication of International Application, Tokuhyo, No. 2003-513092 (published on April 8, 2003; and internationally published on May 10, 2001)
Patent Citation 4
   Japanese Translation Publication of International Application, Tokuhyo, No. 2003-518477 (published on June 10, 2003; and internationally published on May 25, 2001)
Patent Citation 5
   Japanese Translation Publication of International Application, Tokuhyo, No. 2003-519191 (published on June 17, 2003; and internationally published on July 12, 2001)
Patent Citation 6
   Japanese Translation Publication of International Application, Tokuhyo, No. 2003-527391 (published on September 16, 2003; and internationally published on September 20, 2001)
Patent Citation 7
   Japanese Translation Publication of International Application, Tokuhyo, No. 2002-530396 (published on September 17, 2002; and internationally published on June 2, 2000)
Patent Citation 8
   Pamphlet of International Publication No. 03/014727 (internationally published on February 20, 2003)
Patent Citation 9
   Japanese Translation Publication of International Application, Tokuhyo, No. 2002-534384 (published on October 15, 2002)
Patent Citation 10
   Pamphlet of International Publication No. 96/40149 (internationally published on December 19, 1996)
   Cardinal diseases are currently regarded as some of main causes of death in Japan. Thus, there is an urgent demand for the development of a method for preventing or remedying the apoptotic cell death in the cardinal cells caused by the postischemic reperfusion, which causes cardinal infarction, etc. However, as described above, such a drug for preventing or remedying the apoptotic cell death has not been developed.
   In view of this, the present invention utilizes the control of the activity of the Cl- channel that is activated by the apoptosis signal and ROS. An object of the present invention is to provide a method for preventing or remedying the postischemic reperfusion-induced apoptotic cell death in the myocardial cells by using the Cl- channel blocker (inhibitor) and ROS scavenger (eliminator), an inhibitor for inhibiting the apoptotic cell death in the myocardial cells, and a therapeutic drug and preventive drug for diseases caused by the apoptotic cell death, the therapeutic drug and preventive drug containing the inhibitor.

### DISCLOSURE OF INVENTION

The inventors of the present invention diligently studied in order to attain the object. More specifically, using myocardial cells of new-bourn mouse in the primary culture, the inventors, studied (a) whether or not administration of Cl- channel blocker could inhibit the postischemic reperfusion myocardial apoptosis, (b) if could, whether the administration should be done while the cells are ischemic or in reperfusion, and (c) whether or not administration of ROS scavenger can inhibit the postischemic reperfusion-induced apoptosis in the myocardial cells.

The inventors firstly contrived and developed a system for evaluating the above-mentioned points. There has been no appropriate system for evaluating the inhibitive effect on reperfusion-induced cell death. Therefore, it has been impossible to find whether a drug should be administered while the cells are ischemic or in reperfusion. The present invention is accomplished based on the development of the system for evaluation.

In order to attain the object, a method according to the present invention for inhibiting postischemic cell death includes administrating to cells a Cl- channel blocker or ROS scavenger during reperfusion.

Moreover, in the method, the reperfusion-induced cell death may be apoptotic cell death.

The studies conduced by the inventors of the present invention demonstrated that the Cl- channel blocker or the ROS scavenger remedies the apoptotic cell death in incubated mouse myocardial cells damaged by postischemic reperfusion, and the administration of the Cl- channel blocker or the ROS scavenger is effective only when the administration is carried out while the cells are reperfused, but not when the cells are ischemic. The method according to the present invention, arranged as above, can effectively inhibit the cell death caused in postischemic reperfusion, especially apoptotic cell death.

The method according to the present invention may be preferably arranged such that the reperfusion-induced cell death is a cell death that is accompanied by reduction in activity of mitochondria dehydrogenase. This means that the present invention can evaluate the cell death by using the mitochondrial dehydrogenase activity as an indicator.

The method may be preferably arranged such that the reperfusion-induced cell death is a cell death that is accompanied by intracellular DNA fragmentation. This means that the present invention can evaluate the cell death by using the intracellular DNA fragmentation as an indicator.

The method may be preferably arranged such that the reperfusion-induced cell death is a cell death that is accompanied by activation of intracellular protease (caspase) that induces cell death. This means that the present invention can evaluate the cell death by utilizing, as an indicator, the activation of intracellular protease that induces cell death.

The method may be preferably arranged such that the Cl-channel blocker is a VSOR Cl- channel blocker.

The method may be preferably arranged such that the VSOR Cl- channel blocker is 4,4'-diisothiocyanostilbene-2,2'-disulfonic acid (DIDS).

The method may be preferably arranged such that the VSOR Cl- channel blocker is 5-nitro-2-(3-phenylpropylamino)-benzoate (NPPB).

By using, as the Cl- channel blocker, a VSOR Cl- channel blocker such as DIDS, NPPB, or the like, it is possible to effectively inhibit the cell death caused in postischemic reperfusion, especially apoptotic cell death.

The method may be preferably arranged such that the ROS scavenger is 5-dihydroxy-1, 2-benzene disulfonic acid (Tiron).

The method may be preferably arranged such that the ROS scavenger is catalase.

The method may be preferably arranged such that the ROS scavenger is N-acetyl-cysteine (NAC).

By using, as the ROS scavenger, Tiron, catalase, or NAC, it is possible to effectively inhibit the cell death caused in postischemic reperfusion, especially apoptotic cell death.

It is preferable in the method that the cells be myocardial cells. The present invention is applicable to myocardial cells to effectively inhibit myocardial cell death caused in heart transplantation, myocardial cell death caused in heart diseases such as heart infarction, etc. Thus, the present invention is effective in therapies for the heart diseases.

Meanwhile, in order to attain the object, an inhibitor according to the present invention for inhibiting reperfusion-induced cell death contains an ROS scavenger.

Moreover, in order to attain the object, an inhibitor for inhibiting reperfusion-induced cell death contains a Cl- channel blocker.

In order to attain the object, the inhibitor may be arranged such that the Cl- channel blocker is a VSOR Cl-channel blocker.

In the inhibitors, it may be such that the reperfusion-induced cell death is apoptotic cell death.

The inventors of the present invention found that the activation of the Cl- channel caused in cell death (i.e., reperfusion-induce cell death) such as apoptosis is caused by ROS. Thus, the elimination of ROS from the cells by using a ROS scavenger (eliminator) will hinder the activation of the Cl-channel thereby to make it possible to control the reperfusion-induced cell death.

Moreover, the use of a Cl- channel blocker (for example, a VSOR Cl- channel blocker such as DIDS, NPPB, etc.) can effectively inhibit the reperfusion-induced cell death.

The inhibitor may be preferably arranged such that the reperfusion-induced cell death is a cell death that is accompanied by reduction in activity of mitochondria dehydrogenase. This means that the present invention can evaluate the cell death by using the mitochondrial dehydrogenase activity as an indicator.

Moreover, the inhibitor may be preferably arranged such that the reperfusion-induced cell death is a cell death that is accompanied by intranuclear DNA fragmentation (DNA ladder formation). This means that the present invention can evaluate the cell death by using the intracellular DNA fragmentation as an indicator.

The inhibitor may be preferably arranged such that the reperfusion-induced cell death is a cell death that is accompanied by activation of intracellular protease (caspase) that induces cell death. This means that the present invention can evaluate the cell death by utilizing, as an indicator, the activation of intracellular protease that induces cell death.

The inhibitor may be preferably arranged such that the ROS scavenger is Tiron.

The inhibitor may be preferably arranged such that the ROS scavenger is catalase.

The inhibitor may be preferably arranged such that the ROS scavenger is NAC.

As described above, by using, as the ROS scavenger, Tiron, catalase, or NAC, it is possible to effectively inhibit the cell death caused in postischemic reperfusion, especially apoptotic cell death.

Meanwhile, a drug (therapeutic drug) according to the present invention for remedying a disease caused by reperfusion-induced cell death contains any one of the inhibitor mentioned above.

The therapeutic drug is preferably used in remedying a disease caused by myocardial cell death.

The therapeutic drugs of the arrangement can inhibit the activation of Cl- channel and thereby inhibit the reperfusion-induced cell death. Therefore, the therapeutic drugs are effective as a therapeutic drug for myocardial cell death caused in the heart transplantation, myocardial cell death in the heart diseases such as heart infarction, or the other cell death.

On the other hand, a drug (preventive drug) for preventing a disease caused by reperfusion-induced cell death contains the any one of the inhibitor mentioned above.

The preventive drug is preferably used in preventing a disease caused by myocardial cell death.

The preventive drugs of these arrangements can inhibit the activation of Cl- channel and thereby inhibit the reperfusion-induced cell death. Therefore, the therapeutic drugs are effective as a preventive drug for myocardial cell death caused in the heart transplantation, myocardial cell death in the heart diseases such as heart infarction, or the other cell death.

Meanwhile, a method according to the present invention is a method for evaluating how a material to be tested is effective in inhibiting reperfusion-induced cell death. The method includes: performing ischemic treatment in which cells are anaerobically incubated in a buffer containing no glucose; performing reperfusion treatment in which the cells subjected to the ischemic treatment are aerobically incubated in a culture solution containing glucose; and comparing (a) evaluation on cell death in a case where the material to be tested is added during the ischemic treatment or the reperfusion treatment (the step of performing the ischemic treatment or the step of performing the reperfusion treatment) and (b) evaluation on cell death in a case where the material to be tested is not added during the ischemic treatment or the reperfusion treatment.

The method may be preferably arranged such that the material to be tested is added during the reperfusion treatment.

Even though there has been no appropriate system for evaluating the reperfusion-induced cell death and therefore it has been impossible to evaluate the inhibitive effect on the reperfusion-induced cell death in the material to be tested. According to the method according to the present invention for evaluating how a material to be tested is effective in inhibiting reperfusion-induced cell death, it is possible to perform screening for an inhibitor on the reperfusion-induced cell death, and, a therapeutic drug and preventive drug for diseases caused by the apoptotic cell death.

The method may be preferably arranged such that the material to be tested is a Cl- channel blocker or ROS scavenger.

It is known that the apoptotic cell death is accompanied by the activation of the Cl- channel. Therefore, by evaluating a Cl- channel blocker, which inhibits the Cl- channel activation or a ROS scavenger by the method according to the present invention for evaluating the inhibitive effect on the reperfusion-induced cell death, it is possible to perform the screening for an inhibitor on the reperfusion-induced cell death, and, a therapeutic drug and preventive drug for diseases caused by the apoptotic cell death.

In the method, the cells may be myocardial cells.

In the method, the reperfusion-induced cell death may be apoptotic cell death.

The arrangement makes it possible to evaluate the inhibitory effect on the reperfusion-induced cell death, especially, in the myocardial cells. Thus, The arrangement can make it possible to perform the screening for an inhibitor, a therapeutic drug, a preventive drug, etc. for apoptosis in the myocardial cells in the heart transplantation, heart infarction, etc.

The method is arranged such that the evaluation on the cell death utilizes activity of a mitochondria dehydrogenase as an indicator.

This arrangement makes it possible to more easily perform the evaluation of the reperfusion-induced cell death. Therefore, this arrangement make it possible to perform the screening for (a) an inhibitor for inhibiting the reperfusion cell death, and (b) a therapeutic drug and a preventive drug for diseases caused by the apoptotic cell death.

The method is arranged such that the evaluation on the cell death utilizes intranuclear DNA fragmentation as an indicator.

This arrangement makes it possible to perform the evaluation of the reperfusion-induced cell death more easily. Therefore, this arrangement makes it possible to perform the screening for (a) an inhibitor for inhibiting the reperfusion cell death, and (b) a therapeutic drug and a preventive drug for diseases caused by the apoptotic cell death.

The method is arranged such that the evaluation on the cell death utilizes activity of intracellular protease (caspase) that induces cell death.

This arrangement makes it possible to perform the evaluation of the reperfusion-induced cell death more easily. Thus, this arrangement makes it possible to perform the screening for (a) an inhibitor for inhibiting the reperfusion cell death, and (b) a therapeutic drug and a preventive drug for diseases caused by the apoptotic cell death.

Meanwhile, a kit according to the present invention is a kit for performing the method as described above. The kit includes: cells; a buffer containing no glucose; and an culture solution containing glucose.

In the kit, the cells may be myocardial cells.

The kit makes it possible to perform the evaluation of the cell death more easily. Thus, the kit makes it possible to perform the screening for (a) an inhibitor for inhibiting the reperfusion cell death, and (b) a therapeutic drug and a preventive drug for diseases caused by the apoptotic cell death.

In addition to the arrangement, the kit may include a reagent for performing evaluation on cell survival ratio (e.g., Mitochondrial Dehydrogenase Activity Measurement Method (MTT method). More specifically, examples of the reagent for performing the MTT method encompass tetrazolium salt (2-(2-methoxy-4-nitrophenyl-2H-tetrazolium monosodium salt: Dojindo Laboratories). Moreover, the kit may include a reagent or an apparatus for detecting intracellular DNA fragmentation in the tested cells. Examples of the reagent or apparatus may be a buffer solution (EDTA; Na-N-lauoyl sarcosinate containing RNase and Tris-HCl, pH 7.8), protenase K, agarose gel, ethidium bromide, an electrophoresis apparatus, an UV detector, or the like. Moreover, the kit may include a reagent or kit (e.g., CaspASETM assay system (Promega, Madison, WI)) necessary for measuring the caspase 3/7 activity.

For a fuller understanding of the nature and advantages of the invention, reference should be made to the ensuing detailed description taken in conjunction with the accompanying drawings.

### BRIEF DESCRIPTION OF DRAWINGS

FIG. 1(a) is a view illustrating results of evaluation of cell survival ratio of tested cells subjected to various treatments in Example 1. The evaluation is carried out by an MTT method.
FIG. 1(b) is a view illustrating results of DNA ladder test of the tested celled subjected to various treatment in Example 1.
FIG. 2(a) is a view illustrating cell survival ratio (MTT method) in the case where VSOR Cl- channel block (DIDS) of 50µM or 200µM was added during ischemic treatment.
FIG. 2(b) is a view illustrating results of DNA ladder test in the case where VSOR Cl- channel block (DIDS) of 50µM or 200µM was added during ischemic treatment.
FIG. 2(c) is a view illustrating cell survival ratio (MTT method) in the case where VSOR Cl- channel block (DIDS) of 50µM or 200µM was added during reperfusion treatment.
FIG. 2(d) is a view illustrating results of DNA ladder test in the case where VSOR Cl- channel block (DIDS) of 50µM or 200µM was added during reperfusion treatment.
FIG. 3(a) is a view illustrating cell survival ratio (MTT method) in the case where VSOR Cl- channel block (NPPB) of 50µM or 100µM was added during ischemic treatment.
FIG. 3(b) is a view illustrating results of DNA ladder test in the case where VSOR Cl- channel block (NPPB) of 50µM or 100µM was added during ischemic treatment.
FIG. 3(c) is a view illustrating cell survival ratio (MTT method) in the case where VSOR Cl- channel block (NPPB) of 50µM or 100µM was added during reperfusion treatment.
FIG. 3(d) is a view illustrating results of DNA ladder test in the case where VSOR Cl- channel block (NPPB) of 50µM or 100µM was added during reperfusion treatment.
FIG. 4(a) is a view illustrating results of measurement of caspase 3/7 activity in the tested cells subjected to 6-hour ischemic treatment + 24-hour reperfusion treatment in Example 2.
FIG. 4(b) is a view illustrating results of cell survival ratio evaluation (MTT method) of the tested cells subjected to 6-hour ischemic treatment + 96-hour reperfusion treatment in Example 2.
FIG. 5 is a view illustrating results of measurements of reactive oxygen species (ROS) level of myocardial cells after postischemic reperfusion treatment.
FIG. 6(a) is a view illustrating results of measurement of caspase 3/7 activity in case where 1mM Tiron, 2mM NAC, and 200U/ml catalase were respectively added in the reperfusion treatment in Example 3.
FIG. 6(b) is a view illustrating cell survival ratios (MTT method) in case where 1mM Tiron, 2mM NAC, and 200U/ml catalase were respectively added in the reperfusion treatment in Example 3.

### BEST MODE FOR CARRYING OUT THE INVENTION

The present invention relates to inhibition of cell death, especially, postischemic reperfusion-induced cell death.

Here, "reperfusion-induced cell death" is a cell death caused by reperfusion, in which blood flows into the ischemic cells. It is known that most of such cell death is caused by apoptosis (hereinafter, the cell death caused by apoptosis is referred to as apoptotic cell death where appropriate). Examples of reperfusion-induced cell death includes apoptotic cell death of myocardial cell death that occurs in heart transplantation, apoptotic cell death of myocardial cell death caused by myocardial infarction, etc.

Cell death occurs various parts of living organisms, thereby causing various diseases. Development of a method, inhibitor, or the like for inhibiting such cell death, especially, apoptotic cell death is very beneficial because it can provide a drug for effectively remedying or preventing various diseases caused by the cell death.

As described above, the inventors of the present invention have diligently studied the mechanisms how the cell death, including apoptosis, is caused. The inventors proved that Cl-channel is activated and an activation signal for the Cl- is a reactive oxygen species (ROS) (see Non-Patent Citations 6 and 9, etc.).

Therefore, the apoptotic cell death can be inhibited by administrating, to the cells, (i) a Cl- channel blocker that inhibits the activity of the Cl- channel, or (ii) a drug (ROS scavenger) for removing ROS that acts as the activation signal of the Cl- channel.

The method for inhibiting the reperfusion-induced cell death, and drugs for inhibiting, remedying, or preventing the reperfusion-induced cell death according to the present invention are accomplished based on this concept.

As there has been no method for easily evaluating the reperfusion-induced cell death, the inventors of the present invention first established a system for evaluating the reperfusion-induced cell death.

### [System for Evaluating Reperfusion-Induced Cell Death].

The system established by the inventors of the present inventions for evaluating the reperfusion-induced cell death includes performing an ischemic cell death treatment in which cells to be evaluated are anaerobically incubated in a buffer solution containing no glucose, and performing a reperfusion treatment in which the cells subjected to the ischemic treatment are aerobically incubated in a buffer solution containing glucose. Via these two steps, the cells to be evaluated are experienced the postischemic reperfusion. By evaluating and comparing the cell deaths in terms of survival ratios before and after the postischemic reperfusion, it is possible to evaluate the reperfusion-induced cell death.

The cells to be evaluated are not particularly limited. Myocardial cells, cranial nerve cells, brain glia cells can be evaluated appropriately. By evaluating myocardial cells, it is possible to evaluate reperfusion-induced cell death caused by myocardial infarction etc. By evaluating cranial nerve cells and brain glia cells, it is possible to evaluate reperfusion-induced cell death caused by brain infarction etc. For the sake of easy handling or the other reasons, it is preferable that the cells to be evaluated can be incubated.

The buffer solution used in the ischemic treatment step is not particularly limited, provided that it is a buffer solution that does not contain glucose. For example, this buffer solution may preferably contain 108mM NaCl, 0.5mM MgCl₂, 5.4mM KCl, 1mM CaCl₂, 5mM HEPES, and may be adjusted to pH7.4 by adding NaHCO₃ thereto, as employed in Examples later described.

The anaerobic condition under which the cell incubation is carried out in the ischemic treatment step is oxygen concentration of 1% or less. It is preferable that the cell incubation in the ischemic treatment step be carried out under a condition in which no oxygen is contained (0.01%). Thus, the cell incubation may be carried out under carbonic acid gas, nitrogen gas, or argon gas. For example, the cell incubation was carried out under 95% argon gas and 5% carbonic acid gas in Examples later described. The cell incubation may be carried out by using well-known apparatus and method etc. as appropriate.

The cell incubation in the ischemic treatment step may be carried out at a temperature that is suitable to usual cell incubation. It is preferable that the cell incubation be carried out at a temperature in a range of 28 to 40°C. It is most preferable that the cell incubation be carried out at 37°C.

Moreover, as to incubation period (treatment period), the cell incubation in the ischemic treatment step should be carried out until the cells become ischemic. For example, the incubation period is preferably in a range of 1 to 24 hours, more preferably in a range of 2 to 18 hours, and most preferably in a range of 4 to 12 hours. In Examples later described, the ischemic treatment was carried out for 6 hours.

In the reperfusion treatment step, the cells subjected to the ischemic treatment are incubated aerobically in a culture solution (medium) containing glucose. The culture solution (medium) for use in the reperfusion treatment step is not particularly limited, provided that it contains glucose. Well-known media may be employed as appropriate. Suitable examples of the well-known media include D-MEM medium containing 10% FBS, RPMI medium, MEM medium, EM medium, etc.

The aerobic condition is a condition in which oxygen concentration is 5% or more. The oxygen concentration of the aerobic condition is preferably 10% or more, and most preferably 15% or more. In Example later described, the reperfusion treatment is carried out in 95% air (oxygen approximately 20%) and 5% carbonic acid gas.

As to incubation period in the reperfusion treatment step, the incubation should be performed in a period long enough at least for reperfusion of the ischemic cells. The incubation period in the reperfusion treatment is preferably in a range of 12 hours to 144 hours, more preferably in a range of 24 hours to 120 hours, and most preferably in a range of 48 hours to 96 hours. Moreover, in terms of incubation temperature, this incubation may be performed similarly to the incubation in the ischemic treatment step. In Examples later described, the incubation was carried out at 37°C for 96 hours.

The system for evaluating the reperfusion-induced cell death is applicable to screening for an inhibitor for inhibiting the reperfusion-induced cell death, a drug for remedying or preventing a disease caused by apoptotic cell death. That is, by comparing an evaluation result of cell death in case where a material to be tested is introduced in either of the treatment step (the ischemic treatment step or the reperfusion treatment step) and an evaluation result of cell death in case where the material to be tested is not introduced in either of the treatment step (the ischemic treatment step or the reperfusion treatment step), it is possible to evaluate how effective the material to be tested is to inhibit the reperfusion-induced cell death.

According to studies conducted by the inventors of the present invention, significant inhibitory effect on the reperfusion-induced cell death was observed only when a material to be tested (Cl- channel blocker or ROS scavenger) was added in the buffer solution in the reperfusion treatment step. Therefore, it is preferable that a material to be tested (Cl-channel blocker or ROS scavenger) is added in the buffer solution in the reperfusion treatment step.

The system for evaluating the reperfusion-induced cell death is not particularly limited in terms of a method for evaluating the cell death. For example, the evaluation may be carried out by a method (such as MTT method and MTS method) utilizing an activity of mitochondria dehydrogenase as an indicator, a method (such as DNA ladder test) utilizing intranuclear DNA fragmentation as an indicator, a method utilizing an activity of intracellular protease (caspase) as an indicator, a method utilizing, as an indicator, release of cytochrome c from mitochondria to cytoplasm, a method utilizing, as an indicator, a nuclear morphological change such as nuclear condensation, nuclear fragmentation, or other methods.

In short, the concept of the evaluation on the cell death by utilizing the activity of mitochondria dehydrogenase as the indicator is as follows. It is known that cell death lowers the activity of mitochondria dehydrogenase. Thus, it is possible to judge that cell death ratio is high when the activity of the mitochondria dehydrogenase is lowered.

In short, the concept of the evaluation on the cell death by utilizing the intranuclear DNA fragmentation as the indicator is as follows. Apoptosis cell death causes ladder-like fragmentation of the intranuclear DNA. Thus, it is possible to easily judge the apoptotic cell death by observing the intranuclear DNA by electrophoresis or the like.

In short, the concept of the evaluation on the cell death by utilizing caspase activity as the indicator is as follows. It is known that the apoptosis cell death increases caspase activity. Thus, it is possible to judge that the apoptotic cell death ratio is high when the caspase activity is increased.

In short, the concept of the evaluation on the cell death by utilizing the release of cytochrome c from mitochondria to cytoplasm as the indicator is as follows. The apoptotic cell death causes the release of cytochrome c from mitochondria to cytoplasm. Thus, it is possible to easily judge the apoptotic cell death by measuring an amount of cytochrome c leased.

In short, the concept of the evaluation on the cell death by utilizing the nuclear morphological change as the indicator is as follows. The apoptotic cell death causes nuclear morphological changes such as nuclear condensation, nuclear fragmentation, etc. Thus, it is possible to easily judge the apoptotic cell death by observing the nuclear morphological change by using electron-microscope, or the like.

Examples will specifically describe the method (MTT method) utilizing an activity of mitochondria dehydrogenase as the indicator, the method (DNA ladder test) utilizing intranuclear DNA fragmentation as the indicator, and the method utilizing an activity of intracellular protease (caspase) as the indicator.

It is possible to constitute a "kit according to the present invention for performing a method for evaluating an inhibitory effect on reperfusion-induced cell death" by including: cells necessary for performing the method, which is also of the present invention; a buffer solution containing no glucose; and a culture solution containing glucose. The kit according to the present invention may contain a reagent and/or an apparatus necessary for performing the method according to the present invention for evaluating the inhibitory effect on the reperfusion-induced cell death.

### [Inhibitor and Inhibiting Method according to the Present Invention for Inhibiting Reperfusion-Cell Death]

By using the system for evaluating the reperfusion-induced cell death, the inventors of the present invention studies (a) whether the administration of the Cl-channel blocker could inhibit the reperfusion-induced myocardial cell apoptosis or not, (b) if could, the administration should be done while the cells were ischemic (ischemic treatment) or while the cells were reperfused (reperfusion treatment), and (c) whether the administration of ROS scavenger could also inhibit the reperfusion-induced myocardial apoptosis, or not.

The study showed that VSOR (volume-sensitive outwardly rectifying) Cl- channel blocker and ROS scavenger can significantly inhibit the reperfusion on-induced cell death, that is, the VSOR Cl- channel blocker and ROS scavenger can effectively function as inhibitors for the reperfusion-induced cell death. Further, the inventors of the present invention found that it is preferable that the VSOR Cl- channel blocker or the ROS scavenger be administered to the cells while the reperfusion of the cells is being conducted.

What is meant by the term "Cl- channel" is Cl- channels that are activated by a change in the volume in the cells and act to change the volume. Moreover, what is meant by the term "Cl-channel blocker" is drugs that inhibit the activation of the Cl-channels.

Moreover, what is meant by the term "ROS scavenger" is drugs that can eliminate or scavenge ROS.

Examples of the VSOR C1- channel blocker that can be used in the method according to the present invention for inhibiting the reperfusion-induced cell death encompass 4,4'-diisothiocyanostilbene-2,2'-disulfonic acid (DIDS), 5-nitro-2-(3-phenylpropylamino)-benzoate (NPPB), glibenclamid, nifulumic acid, pholoretin, etc.

Examples of the ROS scavenger encompass 5-dihydroxy-1, 2-benzene disulfonic acid (Tiron), catalase, N-acetyl-cysteine (NAC), etc.

In the use of the VSOR Cl- channel blocker or ROS scavenger for inhibiting the reperfusion-induced cell death, concentration of the VSOR Cl- channel blocker or ROS scavenger is determined according to a kind of the drug to use, a kind and condition of cells to treat, etc., and is not particularly limited. Therefore, the concentration of the VSOR Cl- channel blocker or ROS scavenger is determined as most appropriate.

In case where DIDS is used to inhibit the reperfusion-induced cell death in the system for evaluating the reperfusion-induced cell death in ventricular myocardial cells of new-borne mouse, for example, the concentration of DIDS is preferably in a range of 10µM to 300µM, more preferably in a range of 25µM to 250µM, and most preferably in a range of 50µM to 200µM. In later-described Example, the reperfusion-induced cell death was successfully inhibited by administrating DIDS in 50µM and 200µM to the cells.

Moreover, in the case of NPPB, the concentration of NPPB is preferably in a range of 10µM to 200µM, more preferably in a range of 25µM to 150µM, and most preferably in a range of 50µM to 100µM. In later-described Example, the reperfusion-induced cell death was successfully inhibited by administrating NPPB in 50µM and 100µM to the cells.

Moreover, in the case of Tiron, the concentration of Tiron is preferably in a range of 0.05mM to 10mM, more preferably in a range of 0.1mM to 5mM, and most preferably in a range of 0.5mM to 2mM. In later-described Example, the reperfusion-induced cell death was successfully inhibited by administrating Tiron in 1mM to the cells.

In the case of catalase, the concentration of catalase is preferably in a range of 50U/ml to 300U/ml, more preferably in a range of 100U/ml to 250U/ml, and most preferably in a range of 150U/ml to 200U/ml. In later-described Example, the reperfusion-induced cell death was successfully inhibited by administrating catalase in 200U/ml to the cells. Here, "catalase activity 1U" is an activity of an enzyme capable of catalyzing a reaction for breaking down a substrate (hydrogen peroxide: H₂O₂) to water and oxygen at a rate of 1 µmol (micro mol) per min.

In the case of NAC, the concentration of NAC is preferably in a range of 0.1mM to 10mM, more preferably in a range of 0.5mM to 5mM, and most preferably in a range of 1mM to 2mM. In later-described Example, the reperfusion-induced cell death was successfully inhibited by administrating NAC in 2mM to the cells.

In case where the reperfusion-induced cell is inhibited by using VSOR Cl- channel blocker and ROS scavenger, the administration of the drugs mentioned above may be performed in any way. To make a desirable concentration, the drug may be added into the cell incubation medium or the like in a form of a solution in which the drug is dissolved in a solvent or in a form of powder of the drug.

As to timing of the administration of the VOOR Cl-channel blocker and ROS scavenger (inhibitors for reperfusion-induced cell death), it is preferable as described above that the inhibitor be administrated while the cells are reperfused (being subjected to the reperfusion treatment). As exemplified in the later-described Examples, no significant inhibition on the reperfusion-induced cell death could be attained by administrating the VSOR Cl- channel blocker and ROS scavenger (reperfusion-induced cell death inhibitor) to the cells while the cells were ischemic (being subjected to the ischemic treatment). On the other hand, significant inhibition on the reperfusion-induced cell death could be attained by administrating the VSOR Cl- channel blocker and ROS scavenger (reperfusion-induced cell death inhibitor) to the cells while the cells were reperfused (being subjected to the reperfusion treatment).

In heart transplantation using the VSOR Cl- channel blocker and ROS scavenger (inhibitor for the reperfusion-induced cell death), the inhibitor for the reperfusion-induced cell death may be added, for example, via reflow of bloodstream after cardiovascular suture. The use of the VOSR Cl- channel blocker and ROS scavenger (inhibitor for the reperfusion-induced cell death) in the heart transplantation inhibits the cell death in the transplant heart. This greatly improves graft survival after heart transplantation.

Needless to say, the VSOR Cl- channel blocker and ROS scavenger are effective not only to the inhibition of the reperfusion-induced cell death, but also apoptotic cell deaths of other causes.

### [Therapeutic Drug and Preventive Drug According to the Present Invention]

As described above, the VSOR Cl- channel blocker and ROS scavenger can significantly inhibit the apoptotic cell death such as reperfusion-induced cell death and the like. Thus, it is possible to utilize the VSOR Cl- channel blocker and ROS scavenger as a drug for preventing or remedying a disease caused by cell death (e.g., a disease to which progress of apoptosis relates) in mammals (such as human being, mouse, rat, rabbit, dog, cat, cow, horse, pig, monkey, etc.).

Examples of the disease encompass ischemic diseases (myocardial infraction, brain ischemia, etc.), nerve degeneration disease (Alzheimer disease, Parkinson diseases, etc.), stagnant heart failure, etc. In the present invention, the therapeutic drug and preventive drug according to the present invention are applicable to such diseases that occur solely, in combination, or in complication with another disease. Especially, the therapeutic drug and preventive drug according to the present invention are applicable to prevention or remedy of delayed cell death in ischemic diseases.

The therapeutic drug and preventive drug according to the present invention may contain the VSOR Cl- channel blocker and ROS scavenger per se. But usually, the therapeutic drug and preventive drug according to the present invention may be produced by means of a well-known method for manufacturing a medicinal composition by using the VSOR Cl- channel blocker and ROS scavenger, or a salt thereof, and a pharmacologically-allowable carrier, or the like. More specifically, it is possible to use a medicinal composition prepared by mixing the VSOR Cl- channel blocker and ROS scavenger (or a salt thereof) and a pharmacologically-allowable carrier by an ordinary method.

The pharmacologically-allowable carrier may be made of an organic or inorganic carrier material that can be used as a pharmaceutical material. In case of solid drugs, the pharmacologically-allowable carrier may be added as a excipient, glossing agent, a binding agent, a disintegrant, or the like. In case of liquid drugs, the pharmacologically-allowable carrier may be added as a solvent, a dissolution facilitating agent, a dispersing agent, an isotonic agent, a buffering agent, a soothing agent, or the like.

Examples of the excipient encompass lactose, sucrose, D-mannitol, xylitol, sorbitol, erithritol, starch, crystalline cellulose, etc. Examples of the glossing agent encompass magnesium stearate, calcium stearate, talc, colloid silica, etc.

Moreover, examples of the binding agent encompass α-starch, methyl cellulose, crystalline cellulose, sucrose, D-mannitol, trehalose, dextrin, hydroxypropylcellulose, hydroxypropylmethylcellulose, polyvinylpyrrolidone, etc.

Examples of the disintegrant encompass starch, carboxymethyl cellulose, low-substituted hydroxypropylcellulose, carboxymethylcellulose calcium, croscarmellose sodium, carboxy methyl starch sodium, etc.

Examples of the solvent encompass water for injection, alcohol, propylene glycol, macrogol, sesame oil, cone oil, tricapririn, etc.

Examples of the dissolution facilitating agent encompass polyethylene glycol, propylene glycol, D-mannitol, trehalose, benzyl benzoate, ethanol, trisaminomethane, cholesterol, triethanolamine, sodium carbonate, sodium citrate, etc.

Examples of dispersing agent encompass surfactants such as stearyltriethanolamine, sodium lauryl sulfate, lauryl aminopropionate, lecitin, benzalkonium chloride, benzetonium chloride, glycerin monostearate, etc., and hydrophilic macromolecules such as polyvinyl alcohols, polyvinyl pyrrolidone, sodium carboxymethylcellulose, methyl cellulose, hydroxymethyl cellulose, hydroxyethyl cellulose, hydroxyl propyl cellulose, etc.

Examples of the isotonic agent encompass sodium chloride, glycerin, D-mannitol, etc. Examples of the buffering agent encompass phosphate; acetate, carbonate, citrate, etc.

Examples of the soothing agent encompass benzyl alcohols.

Examples of a preservative encompass paraoxybenzoic esters, chlorobutanol, benzyl alcohols, phenyl alcohols, dehydroacetic acid, sorbic acid, etc.

Example of antioxidant encompass sulfite salt, ascorbic acid, etc.

The VSOR Cl- channel blocker content and ROS scavenger content in the therapeutic drug or the preventive drug according to the present invention is, for example, in a range of approximately 5% by weight to approximately 100% by weight, preferably, in a range of approximately 10% by weight to approximately 80% by weight.

The therapeutic drug and the preventive drug according to the present invention can be produced by a method usually employed in the pharmaceutical technical field. The therapeutic drug and the preventive drug according to the present invention may be in a form of oral drugs such as tablets, capsules (including soft capsules and micro capsules), power, particles, syrup, etc., or in a form of non-oral drugs, such as injection drugs, suppositories, pellets, drops, etc. The therapeutic drug and the preventive drug according to the present invention are low in toxicity and can be administered orally or non-orally.

Administration doses of the therapeutic drug and the preventive drug according to the present invention are dependent on an administration target, administration route, symptom, etc, and may be determined as most appropriate.

Referring to drawing attached herewith, Examples are described below thereby to explain the embodiments of the present invention in more details. Needless to say, the present invention is not limited to the following Examples, and may be modified more details in various ways. Furthermore, the present invention is not limited to the embodiments mentioned above and may be modified in various way within the scope of claims below. The technical scope of the present invention also encompasses embodiments attained by appropriately combining the technical means disclosed herein.

In the following Examples, primary culture of ventricular myocardial cells of new-borne mouse was used to find (1) whether the administration of VSOR Cl- channel blocker could inhibit the reperfusion-induced myocardial cell apoptosis or not, (2) if could, the administration should be done while the cells were ischemic or while the cells were reperfused, and (3) whether the administration of ROS scavenger could also inhibit the reperfusion-induced myocardial apoptosis, or not.

### [Example 1: Whether Apoptotic Death of Mouse Myocardial Cells Could be Remedied by VSOR Cl- Channel Blocker]

The inventors of the present invention have already showed that staurosporine (STS) induced apoptosis in incubated myocardial cells of rats can be remedied by 4,4'-diisothiocyanostilbene-2,2'-disulfonic acid (DIDS), which is a VSOR Cl- channel blocker. (S. Tanabe, E. Maeno & Y. Okada "Prevention of staurosporine-induced apoptotic cell death by DIDS and SITS in rat cardiomyocytes in primary culture." Jpn. J. Physiol. 52 (Suppl), S34, 2002) In this Example, it was studied whether apoptosis of mouse myocardial cells could be remedied by VSOR Cl- channel blocker or not.

### (Method)

### (1-1) Cells and Cell Incubation Method

In the following Examples, primary culture of myocardial cells prepared by enzymic treatment from a heart of a new-borne mouse. More specifically, the primary culture of the myocardial cells was prepared as follows.

A ventricle portion was obtained from a heart enucleated from a new-bourn mouse. The ventricle portion was fragmented and then incubated in a solution of 0.05% trypsin and 10mM EDTA (37°C) for a certain time, thereby obtaining free Myocardial cells. The free myocardial cells were collected by centrifugation. The thus obtained cells were incubated in a collagen-coated plate in a density of 1 to 8 x 105 cells/cm².

Moreover, the incubated mouse myocardial cells were incubated in D-MEM medium (+10% FBS) at 37°C for 72 to 96 hours.

### (1-2) STS treatment

The incubated mouse myocardial cells were subjected to STS treatment to induce apoptosis therein. The STS treatment was carried out in the following manner using 4µM STS (Sigma-Aldrich Co.). D-MEM (+10% FBS) containing STS of a certain concentration was added to the myocardial cells that were being incubated in normal conditions (37%, 5% carbonic acid gas). And then, the myocardial cells were further incubated for a certain period of time.

### (1-3) VSOR Cl- channel blocker

Apoptosis inhibition effect of VSOR Cl- channel blockers, namely, 4,4'-diisothiocyanostilbene-2,2'-disulfonic acid (DIDS, Sigma-Aldrich Co.), and 5-nitro-2-(3-phenylpropylamino)-benzoate(NPPB, Sigma-Aldrich Co.) were examined.

The apoptosis inhibition effect of each VSOR Cl- channel blocker was evaluated by added 250µM DIDS or 100µM NPPB in the STS treatment.

### (1-4) Mitochondrial Dehydrogenase Activity Measurement Method (MTT method)

Cell survival ratio was evaluated by Mitochondrial Dehydrogenase Activity Measurement Method (MTT method), which was conduced as follows. To the medium of 100µl in which the incubated mouse myocardial cells subjected to a predetermined treatment were inoculated, 10µl tetrazolium salt (2-(2-methoxy-4-nitrophenyl-2H-tetrazolium monosodium salt: Dojindo Laboratories) to produce highly sensitive water-soluble formzan was added. After the medium was incubated at 37°C for 4 hours, an amount of formzan produced according to activity of the mitochondria dehydrogenase was measured at absorbance of 450nm.

### (1-5) DNA Ladder Test

DNA nucleosome fragmentation of the cells to be tested was detected by DNA ladder detection method. That is, the cells were decomposed in a dissolving buffer solution (EDTA 10mM, 0.5% Na-N-lauoyl sarcosinate, RNase 500µg/ml, Tris-HCl 50mM, pH 7.8) at 37°C for 1 hour. The decomposed cells were treated with protenase K of 500µg/ml at 37°C for 1 hour. Next, chromosome DNA was analyzed by 2% agarose gel electrophoresis, and stained with ethidium bromide.

### (Result)

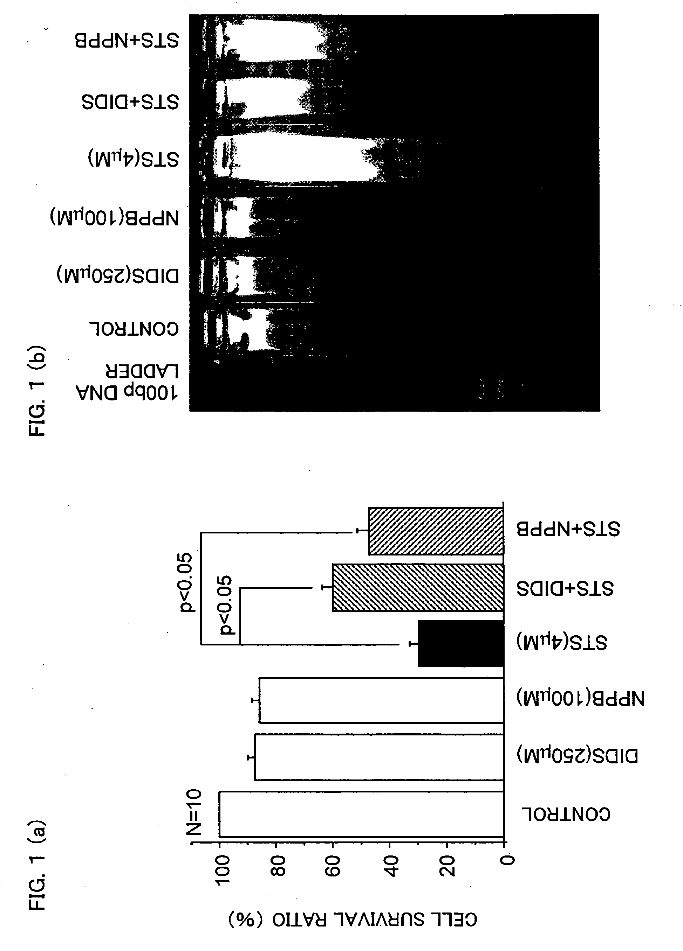

Results are shown in FIGS. 1(a) and 1(b). FIG. 1(a) shows the result of the cell survival ratios of the tested cells treated with various treatments. The cell survival ratios were evaluated by the MTT method. From the left most lane, FIG.1 (a) shows: control (no STS treatment), cells treated with 250µM DIDS ("DIDS (250µM)" in FIG. 1(a)), cells treated with 100µM NPPB ("NPPB (100µM)" in FIG. 1(a)), cells treated with 4µM STS ("STS (4µM)" in FIG. 1(a)), cells treated with 4µM STS + 250µM DIDS ("STS + DIDS" in FIG. 1(a)), cells treated with 4µM STS + 100µM NPPB ("STS + NPPB" in FIG. 1(a)). The cell survival ratio of each case is described in percentage relative to the cell survival ratio of the control which is put as "100%". In FIG. 1(a), some graphs were connected each other by a line labeled as "p < 0.05". This indicates that there was a significant difference between the data shown by the bars at a percentage of risk of less than 5%.

Moreover, FIG. 1(b) shows the results of the DNA ladder test of the tested cells subjected to the treatments. From the left most lane, FIG.1(b) shows: control (no STS treatment, cells treated with 250µM DIDS ("DIDS (250µM)" in FIG. 1(b)), cells treated with 100µM NPPB ("NPPB (100µM)" in FIG. 1(b)), cells treated with 4µM STS ("STS (4µM)" in FIG. 1(b)), cells treated with 4µM STS + 250µM DIDS ("STS + DIDS" in FIG. 1(b)), and cells treated with 4µM STS + 100µM NPPB ("STS + NPPB" in FIG. 1(b)).

As shown in FIG. 1(a), the cell survival ratio was reduced to 29.8% when the cells were treated with 4µM STS for 8 hours. DNA of the cells were degrade into ladder-like fragments, which is characteristic in the apoptotic death. (See FIGS. 1(a) and 1(b) "STS (4µM)")

On the other hand, the cell survival ratio was increased to 59.7% by treating the cells with STS and DIDS (250µM), which is a VSOR Cl- channel blocker. The cells showed no DNA ladder. (See FIGS. 1(a) and 1(b) "STS + DIDS") Similarly, NPPB (100µM), which is also a VSOR Cl- channel blocker, increased the cell survival ratio to 47.2%, showing no DNA ladder. (See FIGS. 1(a) and 1(b) "STS + NPPB")

These results showed that DIDS and NPPB, which are VSOR Cl- channel blockers, remedy the apoptosis in the incubated mouse myocardial cells.

### [Example 2: Whether VSOR Cl- Channel Blockers Remedy Myocardial Cell Apoptosis in Cells Damaged by Postischemic Reperfusion]

### (Postischemic Reperfusion Test)

External solution around the incubated mouse myocardial cells was replaced with a solution containing no glucose (containing 108mM NaCl, 0.5mM MgCl₂, 5.4mM KCl, 1mM CaCl₂, 5mM HEPES, and being adjusted to pH 7.4 with NaHCO₃). Then, the incubated mouse myocardial cells was incubated at 37°C for 6 hours anaerobically (95% argon gas and 5% carbonic acid gas) for ischemic treatment (oxygen glucose deprivation treatment).

Immediately after that, the solution was replaced with an ordinary culture solution containing glucose (D-MEM solution + 10% FBS) and incubated aerobically (95% air + 5% carbonic acid gas) at 37°C for 96 hours for reperfusion treatment (glucose re-administration and reoxygenation).

### (Caspase 3/7 Activity Measurement)

Fluorescent measurement assay was used to measure activity of caspase 3/7, which is a characteristic phenomenon in the apoptotic cell death. In order to exclude influence from other protease related thereto, fluorescent difference in absent or present of a specific inhibitor factor for the caspase 3/7. Caspase 3/7 (Ac-DEVD-AMC), and a tetra peptide inhibiting factor (Ac-DEVD-CHO) of the caspase 3/7 were analyzed by CaspaSETM assay system (Promega, Madison, WI) using a fluorescent material labeled with fluorescent pigment 7-amino 4-methyl coumarin (AMC).

### (Result)

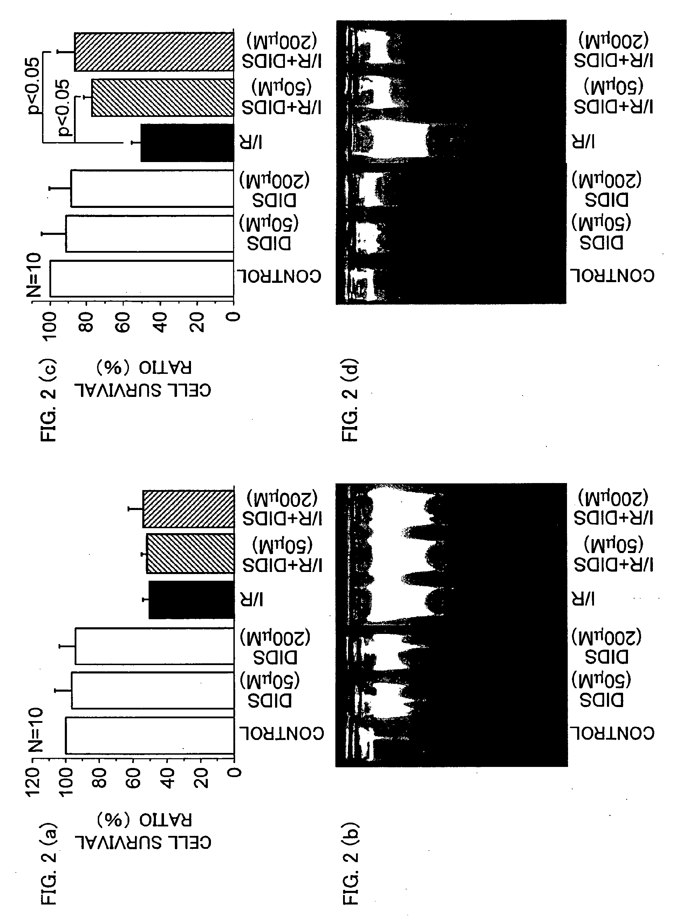

FIGS. 2(a) and 2(b) show cell survival ratios (MTT method) and results of DNA ladder test of cells to which 50µm or 200µm VSOR Cl- channel blocker (DIDS) was added during the ischemic treatment. Moreover, FIGS. 2(c) and 2(d) show cell survival ratios (MTT method) and results of DNA ladder test of cells to which 50µM or 200µM VSOR Cl- channel blocker (DIDS) was added during the reperfusion treatment. FIGS. 2(a) to 2(d), from the left most, show results of control (no postischemic reperfusion treatment), cells treated with 50µM DIDS ("DIDS (50µM)" in the figures), cells treated with 200µM DIDS ("DIDS (200µM)" in the figures), cells with postischemic reperfusion treatment ("I/R" in the figures), cells treated with 50µM DIDS during the ischemic treatment or the reperfusion treatment ("I/R + DIDS (50µM)" in the figures), and cells treated with 200µM DIDS during the ischemic treatment or the reperfusion treatment ("I/R + DIDS (200µM)" in the figures).

The cell survival ratios in FIGS. 2(b) and 2(d) are shown in percentage where the cell survival ratio of the control is put as 100%. Moreover, some bars connected with each other by a line labeled as "p< 0.05" are shown in FIGS. 2(b) and 2(d), indicating that there was a significant difference between data of the two bars at a percentage of risk of less than 5%.

As shown in FIGS. 2, 96-hour reperfusion (R) following 6-hour ischemia (I) reduced the cell survival ratio to 50.2% (see "I/R" in FIGS. 2(a) and 2(c)). The cells in this case showed characteristic DNA ladder in the apoptosis (see "I/R" in FIGS. 2(b) and 2(d)).

The administration of VSOR C1- channel blocker DIDS (50 or 200µM) during the ischemic treatment could not achieve significant inhibition on the apoptosis cell death in this case (see "I/R + DIDS (50µM)" and "I/R + DIDS (200µM)" in FIGS. 2(a) and 2(b)). Meanwhile, administration of VSOR Cl- channel blocker DIDS during the reperfusion treatment could significantly inhibit the apoptosis cell death (see "I/R + DIDS (50µM)" and "I/R + DIDS (200µM)" in FIGS. 2(c) and 2(d)).

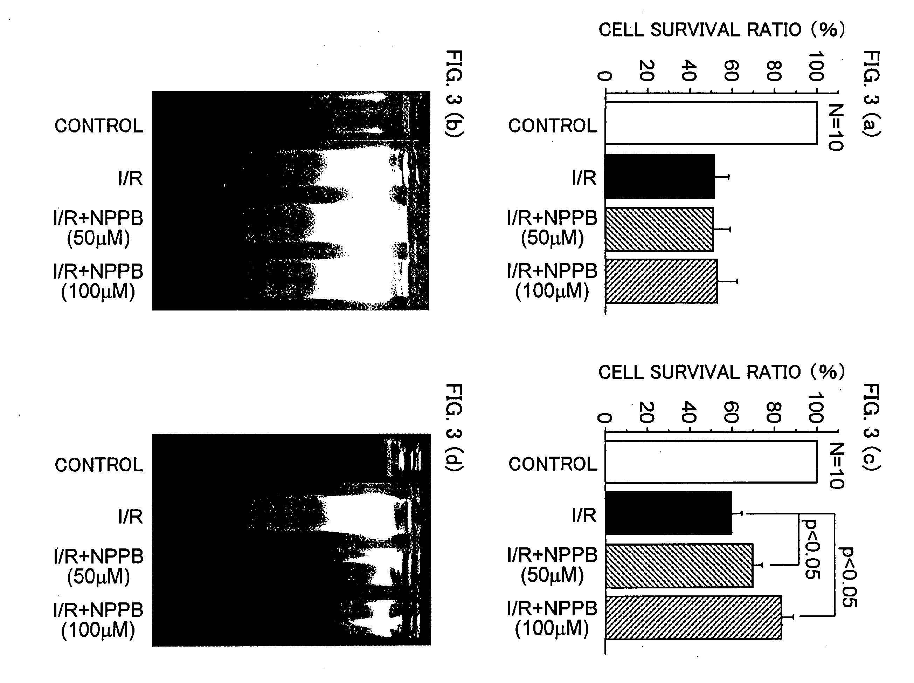

Similar tests were conducted with NPPB. The results of the tests are shown in FIGS. 3(a) to 3(d). FIGS. 3(a) and 3(b) show the cell survival ratios (MTT method) and results of DNA ladder test of cells to which with VSOR C1- channel blocker (NPPB) was added in 50µM or 100µM during the ischemic treatment. Moreover, FIGS. 3(c) and 3(d) show cell survival ratios (MTT method) and results of DNA ladder test of cells to which 50µM or 100µM VSOR Cl- channel blocker (NPPB) was added during the reperfusion treatment. FIGS. 3(a) to 3(d), from the left most, show results of control (no postischemic reperfusion treatment), cells subjected postischemic reperfusion ("I/R" in the figures), cells treated with 50µM NPPB during the ischemic treatment or during the reperfusion treatment ("I/R + NPPB (50µM)" in the figures), and cells treated with 100µM NPPB during the ischemic treatment or during the reperfusion treatment ("I/R + NPPB (100µM)" in the figures). The cell survival ratios in FIGS. 3(a) and 3(c) are shown in percentage relative to the cell survival ratio of the control which is put as 100%. Moreover, some bars connected with each other by a line labeled as "p< 0.05" are shown in FIGS. 3(a) and 3(c), indicating that there was a significant difference between data of the two bars at a percentage of risk of less than 5%.

According to FIG. 3, the administration of NPPB resulted same as that of DIDS. This showed that the VSOR Cl- channel blockers, DIDS and NPPB remedy the apoptotic cell death in the incubated mouse myocardial cells damaged by ischemia and reperfusion, and the administration of any of the VSOR Cl-channel blockers is effective when it is administrated while the cells are reperfused but not while the cells are ischemic.

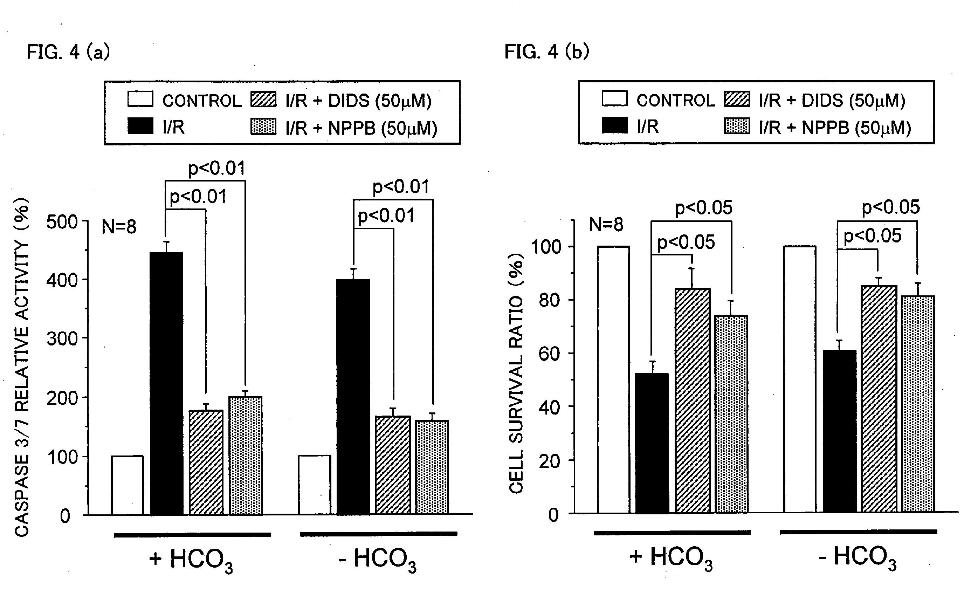

FIG. 4(a) shows results of measurement of caspase 3/7 activity that is characteristic in the apoptotic cell death. FIG. 4(a) shows results of measurement of the caspase 3/7 activity in cells to which 50µM DIDS or 50µM NPPB was added during the reperfusion treatment (see bar graph labeled as "+HCO₃" in FIG. 4(a)). FIG. 4(a) also shows the results for the cells treated with reperfusion treatment using a reperfusion liquid having no HCO₃ (see bar graph labeled as "-HCO₃" in FIG. 4(a)). In the bar graph labeled as "+HCO₃" in FIG. 4(a), the bars respectively show results of control (no postischemic reperfusion), cells treated with postischemic reperfusion ("I/R" in FIG. 4(a)), cells treated with 50µM NPPBduring the reperfusion treatment "I/R + DIDS (50µM)", and cells treated with 50µM NPPB during the reperfusion treatment "I/R + NPPB (50µM)". In the bar graph labeled as "+HCO₃" in FIG. 4(b) and bar graph labeled as "-HCO₃" in FIG. 4(b), the bars are aligned in the same way as in FIG. 4(a).

The relative activities of caspase 3/7 in each case in FIGS 4.(a) etc. are shown in percentage relative to that of the control which is put as 100%. The cell survival ratios of each case in FIGS. 4(b) are shown in percentage relative to that of the control which is put as 100%. In FIG. 4(a), some bars are connected with each other by a line labeled with "p< 0.01". This indicates that there was a significant difference between the data of the two bars at a percentage of risk of less than 1%. In FIG. 4(b), the bar graphs are connected with a line labeled with "p< 0.05". This indicates that there was a significant difference between the data of the two bars at a percentage of risk of less than 5%.

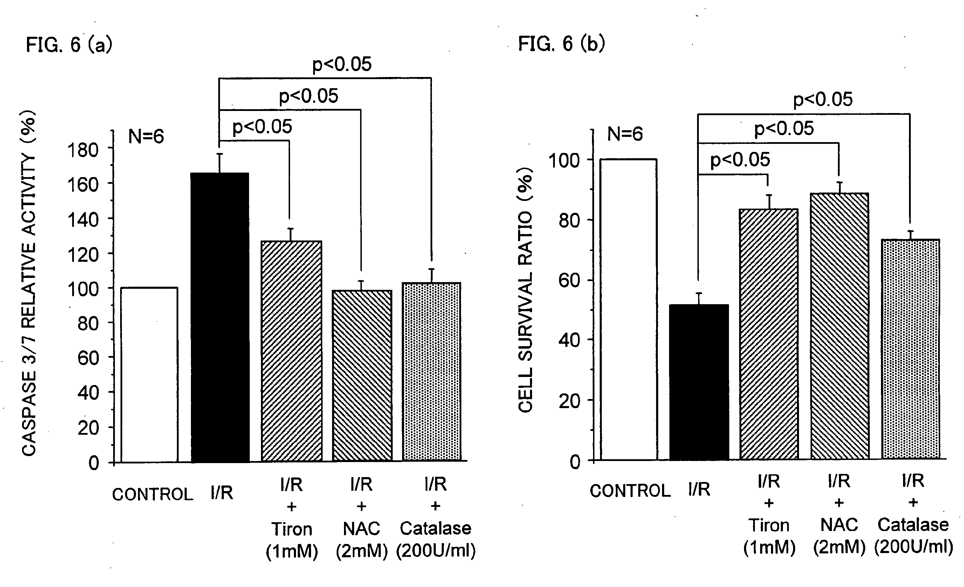

According to FIG. 4(a), 6-hour ischemic treatment + 24-hour reperfusion treatment increased the caspase 3/7 activity in the cells (see "I/R" in FIG. 4(a)). It was found that this increase in the caspase 3/7 activity could be significantly inhibited by administrating 50µM DIDS or 50µM NPPB during the reperfusion treatment (see "I/R + DIDS (50µM)" and "I/R + NPPB (50µM)" in FIG. 4(a)).

The caspase 3/7 activity was also increased by the postischemic reperfusion treatment with the reperfusion liquid (adjusted in pH with Tris) having no NaHCO₃ (see "I/R" in FIG. 4(a)). Further, the increase in the caspase 3/7 activity was also significantly inhibited by the administration of 50µM DIDS or 50µM NPPB during the reperfusion treatment (see "I/R + DIDS (50µM)" and "I/R + NPPB (50µM)" in FIG. 4(a)).

The similar results were obtained in the cell survival ratio test (MTT method) of the cells treated with 6-hour ischemic treatment + 96-hour reperfusion treatment (see FIG. 4(b)).

These results demonstrated that DIDS was effective even under the conditions where Cl-/HCO₃- antiporter (anion exchanger: AE) and Na⁺-HCO₃- symporter (Na⁺-bicarbonate cotransporter: NBC) did not function so actively due to the lack of HCO₃-. Therefore, it was found that DIDS, which is also known as an AE blocker and an NBC blocker, acted as a Cl-channel blocker in this case.

### [Example 3: Whether ROS Scavenger Remedy Myocardial Cell Apoptosis in Cells Damaged by Postischemic Reperfusion]

The inventors of the present invention proved that Apoptosis induction in HeLa cells by STS increases ROS production, resulting in activation of the VSOR Cl- channel thereby to cause AVD, and that the administration of ROS scavenger significantly inhibits the activation of the VSOR Cl-channel, AVD, the increase in the caspase 3/7 activity, and the cell death (see Non-Patent Citation 9). Therefore, by DCF fluorescent method (Non-Patent Citation 9), it was studied whether the myocardial cells produced ROS while they were ischemic or while they were reperfused.

The DCF fluorescent measurement method was carried out as follows. Incubated mouse myocardial cells subjected to the prescribed ischemic treatment and reperfusion treatment were incubated for 30 minutes in a culture solution containing 10µM DCF. DCF reacted with ROS thereby to produce oxidated DCF, which emits 538nm fluorescence when exited with 485nm wavelength. This test measured 538nm fluorescence caused by the oxidated DCF in the solution radiated with 485nm wavelength.

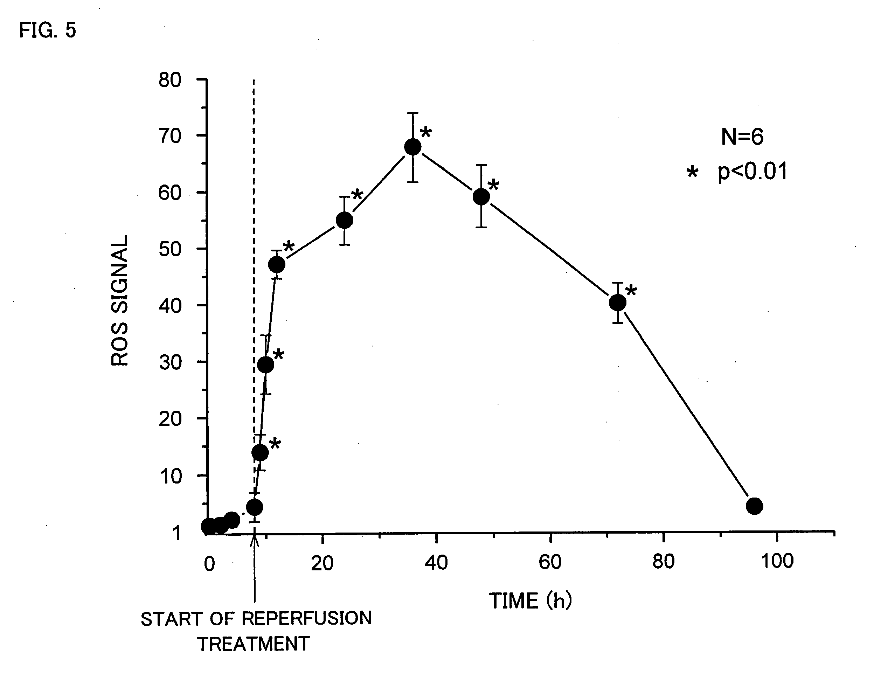

Results of the test are shown in FIG. 5. As shown in FIG. 5, the ROS level in the incubated mouse myocardial cells rapidly increased immediately after the start of the reperfusion treatment. The ROS peaked at 27 hours from the start of the reperfusion treatment (that is, 36 hours later in total), and then gradually returned to its original level at 96 hours later. In FIG. 5, the symbol "*" indicates that the data to which the symbol "*" was attached had a significant difference in the ROS level in the incubated mouse myocardial cells in ischemia at a percentage of risk of less than 1% (p < 0.01).

Next, it was tested whether the administration of the ROS scavenger during the reperfusion treatment could remedy the apoptosis death of the incubated mouse myocardial cells or not. Three ROS scavengers were used in the test, namely, 4, 5-dihydroxy-1, 2-benzene disulfonic acid (Tiron, made by Wako Pure Chemical Industries Ltd.), catalase (made by Sigma-Aldrich Co.), and N-acetyl-cysteine (NAC, made by Sigma-Aldrich Co.). Tiron is a scavenger for superoxide anion. Catalase is a scavenger for hydrogen peroxide. NAC is a non-specific ROS scavenger.

Results are shown in FIGS. 6. FIG. 6(a) shows the results of the measurements of the caspase 3/7 activities in the cells to which 1mM Tiron, 2mM NAC, and 200U/ml catalase were respectively added. From its left most, FIG. 6(a) shows the results of control (no postischemic reperfusion treatment), cells subjected to the postischemic reperfusion treatment ("I/R" in FIG. 6(a)), cells treated with 1mM Tiron in the reperfusion treatment ("I/R + Tiron (1mM) in FIG. 6(a)), cells treated with 2mM NAC in the reperfusion treatment (I/R + NAC (2mM) in FIG. 6(a)), cells treated with 200U/ml catalase in the reperfusion treatment ("I/R + Catalase (200U/ml)" in FIG. 6(a)). FIG. 6(b) shows cell survival ratios (MTT method) of the same cases, which are aligned in the same way as in FIG. 6(a).

In FIG. 6(a), the caspase 3/7 relative activities of each case are shown in percentage (%) relative to that of the control which is put as 100%. In FIG. 6(b), the cell survival ratios of each case are shown in percentage (%) relative to that of the control which is put as 100%. In FIGS. 6(a) and 6(b), some bars are connected with each other by a line labeled as "p<0.05", indicating that there was a significant difference between the data at percentage of risk of less than 5%.

As shown in FIG. 6, it was found that Tiron (1mM), NAC (2mM), and catalase (200U/ml) significantly inhibited the increase in the caspase 3/7 activity (see FIG. 6(a)) and the cell death (see FIG. 6(b)), which would be caused by the postischemic reperfusion treatment (see FIGS. 6(a) and 6(b)). This indicates that the postischemic reperfusion-induced apoptosis in the incubated mouse myocardial cells can be remedied by eliminating the ROS produced in the reperfusion treatment.

Therefore, it was proved that the administration of the ROS scavenger (Tiron, NAC, catalase) to the cells in the reperfusion treatment can significantly inhibit the apoptotic cell death.

### Industrial Applicability

A method according to the present invention for evaluating an inhibitory effect of reperfusion-induced cell death evaluates apoptotic death in myocardial cells in a postischemic reperfusion model test in which the myocardial cells are subjected to oxygen glucose deprivation treatment (ischemic treatment step) and then glucose reintroduction and reoxygenation (reperfusion treatment). The method according to the present invention evaluates the apoptotic death in the myocardial cells by caspase activity measurement, DNA ladder monitoring, or cell survival ratio measurement. It becomes possible to perform screening for therapeutic drugs or preventive drugs for the myocardial cell apoptotic death in myocardial infarction and heart transplantation by evaluating these drugs by the method according to the present invention for evaluating the inhibitory effect of reperfusion-induced cell death.

By using the method according to the present invention for evaluating the inhibitory effect of reperfusion-induced cell death, it was showed that the postischemic reperfusion-induced apoptotic death in the myocardial cells can be inhibited by the administration of a Cl- channel blocker or an ROS scavenger during the reperfusion. Thus, it was found that the Cl- channel blocker or the ROS scavenger can be used as a reperfusion-induced cell death inhibitor, and a therapeutic drug and a preventive drug for diseases caused by the reperfusion-induced cell death.

It is considered that inhibition on the activation of the VSOR Cl- channel that will cause apoptotic volume decrease (AVD) in the initial stage of the apoptosis achieves this remedying mechanism. The present invention provides prevention of the postischemic myocardial damages by inhibiting the VSOR Cl- channel activation, which occurs in the most initial stage of the apoptosis, and an activation signal of the VSOR Cl- channel activation. Therefore, the present invention is utterly novel.

The present invention can be relatively easily applied to clinical treatment, because the present invention works by administrating the Cl- channel blocker or the ROS scavenger not in the ischemic insult, but in the reperfusion treatment after the ischemic insult. The present invention can be employed during the post-transplantation reperfusion in heart transplantation, in which postischemic reperfusion inevitably causes damages. The use of the present invention increases graft survival of the heart plantation.

As described above, the present invention can provide an effective method for inhibiting reperfusion-induced cell death, cell death inhibitor, a drug for remedying or preventing various diseases caused by the reperfusion-induced cell death, etc. Furthermore, the present invention provides a method for evaluating an inhibitory effect on the reperfusion-induced cell death.

Therefore, the present invention is widely applicable to medical industries such as pharmaceutical industry. The present invention also contributes to fundamental research on the reperfusion-induced cell death.

## Claims

1. A method for evaluating how a material to be tested is effective in inhibiting reperfusion-induced cell death, the method comprising:
performing ischemic treatment in which cells are anaerobically incubated in a buffer containing no glucose;
performing reperfusion treatment in which the cells subjected to the ischemic treatment are aerobically incubated in a culture solution containing glucose; and
comparing (a) evaluation on cell death in a case where the material to be tested is added during the ischemic treatment or the reperfusion treatment and (b) evaluation on cell death in a case where the material to be tested is not added during the ischemic treatment or the reperfusion treatment.

2. The method as set forth in claim 1, wherein the material to be tested is added during the reperfusion treatment.

3. The method as set forth in claim 1 or 2, wherein the material to be tested is a C1- channel blocker or ROS scavenger.

4. The method as set forth in any one of claims 1 to 3, wherein the cells are myocardial cells.

5. The method as set forth in any one of claims 1 to 4, wherein the reperfusion-induced cell death is apoptotic cell death.

6. The method as set forth in any one of claims 1 to 5, wherein the evaluation on the cell death utilizes activity of a mitochondria dehydrogenase as an indicator.

7. The method as set forth in any one of claims 1 to 5, wherein the evaluation on the cell death utilizes intranuclear DNA fragmentation as an indicator.

8. The method as set forth in any one of claims 1 to 5, wherein the evaluation on the cell death utilizes activity of intracellular protease that induces cell death.

9. A kit for performing the method as set forth in any one of claims 1 to 8, the kit comprising:
cells;
a buffer containing no glucose; and
a culture solution containing glucose.

10. The kit as set forth in claim 9, wherein the cells are myocardial cells.

11. A method for inhibiting reperfusion-induced cell death, comprising:
administrating to ischemic cells a Cl- channel blocker or ROS scavenger during reperfusion.

12. The method as set forth in claim 11, wherein the reperfusion-induced cell death is apoptotic cell death.

13. The method as set forth in claim 11 or 12, wherein the reperfusion-induced cell death is a cell death that is accompanied by reduction in activity of mitochondria dehydrogenase enzyme.

14. The method as set forth in claim 11 or 12 wherein the reperfusion-induced cell death is a cell death that is accompanied by intracellular DNA fragmentation.

15. The method as set forth in claim 11 or 12 wherein the reperfusion-induced cell death is a cell death that is accompanied by activation of intracellular protease that induces cell death.

16. The method as set forth in any one of claims 11 to 15, wherein the Cl- channel blocker is a VSOR Cl- channel blocker.

17. The method as set forth in claim 16, wherein the VSOR Cl- channel blocker is DIDS.

18. The method as set forth in claim 16, wherein the VSOR Cl- channel blocker is NPPB.

19. The method as set forth in any one of claims 11 to 15 wherein the ROS scavenger is Tiron.

20. The method as set forth in any one of claims 11 to 15, wherein the ROS scavenger is catalase.

21. The method as set forth in any one of claims 11 to 15, wherein the ROS scavenger is NAC.

22. The method as set forth in any one of claims 11 to 21, wherein the cells are myocardial cells.

23. An inhibitor for inhibiting reperfusion-induced cell death, the inhibitor comprising an ROS scavenger.

24. An inhibitor for inhibiting reperfusion-induced cell death, the inhibitor comprising a Cl- channel blocker.

25. The inhibitor as set forth in claim 24 wherein the Cl-channel blocker is a VSOR Cl- channel blocker.

26. The inhibitor as set forth in any one of claims 23 to 25, wherein the reperfusion-induced cell death is apoptotic cell death.

27. The inhibitor as set forth in any one of claims 23 to 26, wherein the reperfusion-induced cell death is a cell death that is accompanied by reduction in activity of mitochondria dehydrogenase enzyme.

28. The inhibitor as set forth in any one of claims 23 to 26, wherein the reperfusion-induced cell death is a cell death that is accompanied by intracellular DNA fragmentation.

29. The inhibitor as set forth in any one of claims 23 to 26, wherein the reperfusion-induced cell death is a cell death that is accompanied by activation of intracellular protease that induces cell death.

30. The inhibitor as set forth in any one of claims 23, 26, 27, 28, or 29, wherein the ROS scavenger is Tiron.

31. The inhibitor as set forth in any one of claims 23, 26, 27, 28, or 29, wherein the ROS scavenger is catalase.

32. The inhibitor as set forth in any one of claims 23, 26, 27, 28, or 29, wherein the ROS scavenger is NAC.

33. A drug for remedying a disease caused by reperfusion-induced cell death, the drug comprising the inhibitor as set forth in any one of claims 23 to 32.

34. The drug as set forth in claim 33, wherein the drug is used in remedying a disease caused by myocardial cell death.

35. A drug for preventing a disease caused by reperfusion-induced cell death, the drug comprising the inhibitor as set forth in any one of claims 23 to 32.

36. The drug as set forth in claim 35, wherein the drug is used in preventing a disease caused by myocardial cell death.
